# EUROPEAN PATENT SPECIFICATION

(11) **EP 2 210 103 B1**
(45) Date of publication and mention of the grant of the patent: **04.03.2015**
(21) Application number: 08850523.5
(22) Date of filing: 12.11.2008
(51) Int. Cl.: G01N 33/564

(54) **METHOD FOR THE IMMOBILIZATION OF A CAPTURE MOLECULE ON A SOLID SUPPORT**
VERFAHREN ZUR IMMOBILISIERUNG EINES ANALYTS AUF EINEM FESTEN TRÄGER
PROCÉDÉ POUR L'IMMOBILISATION D'UN ANALYTE SUR UN SUPPORT SOLIDE

(30) Priority: 12.11.2007 EP 07021878
(43) Date of publication of application: 28.07.2010
(73) Proprietor: Eurodiagnostica AB, 202 11 Malmö (SE)
(72) Inventor: BOEKEL, VAN, Martinus, Adrianus, Maria, NL-5388 CN Nistelrode (NL); VERHEIJEN, Ronald, NL-6611 CE Overasselt (NL); SALDEN, Martinus, Hubertus, Leonardus, NL-6536 CL Nijmegen (NL)
(74) Representative: Habets, Winand
(86) International application number: PCT/EP2008/065394
(87) International publication number: WO 2009/062959

(56) References cited:
- WO-A-2005/116651
- WO-A-2007/017556
- WO-A-2007/039280
- US-A1- 2007 148 704
- KOIVULA MARJA-KAISA ET AL: "SENSITIVE IMMUNOASSAYS FOR THE AUTOANTIBODIES REACTING AGAINST CITRULLINATED CARBOXY-TERMINAL TELOPEPTIDES OF TYPE I AND TYPE II COLLAGENS IN PATIENTS WITH RHEUMATOID ARTHRITIS" CLINICAL CHEMISTRY AND LABORATORY MEDICINE, WALTER DE GRUYTER UND CO, DE, vol. 43, no. 12, December 2005 (2005-12), pages 1400-1405, XP008072567 ISSN: 1434-6621

## Description

### Field of the invention

This invention is in the field of diagnostic assays, more in particular immunoassays wherein a capture molecule is immobilized on a solid support in order to capture an analyte.

### Background art

One of the main goals of investigators in the fields of (clinical) diagnostics and contamination control programs consists of providing new analytical strategies to obtain accurate data as fast as possible. When a new screening test is required for commercial purposes, the method has to be sensitive, specific, fast, cheap and easy to perform. In general, immunological techniques (immunoassays) can fulfill these requirements to a great extent.

Over the past decade, single-use lateral flow immunoassays have been extremely successful both in the laboratory, outpatient clinic and primary care environments. In this type of assay, typically all reaction components may be impregnated or immobilised on a porous solid phase, usually a nitrocellulose membrane, and are brought into contact with the sample, optionally after the addition of a diluent (Zuk RF, Ginsberg VK, Houts T et al. (1985) Clin Chem 31: 1144-1150, Bunce RA, Thorpe GH, Keen L (1991) Anal Chim Acta 249: 263-269, and May K (1994) In: D Wild (ed): The Immunoassay Handbook. Macmillan Press, London, 233-235).

This immunoassay format is also known as strip test, one step strip test, immunochromatographic test, rapid flow diagnostic, rapid immunoassay (test), lateral flow immunoassay (LFI), on site test (assay) or near patient test (NPT).

Immunoassays are analytical measurement systems that rely on the binding between antigens and antibodies for the detection of specific analytes in samples such as for instance clinical samples.

In a typical immunoassay, antigens or antibodies are usually attached to some kind of label and are then used as a detection reagent to detect the analyte. A detection reagent such as an antibody or antigen attached to a label is usually referred to as a conjugate. The label may for instance be a radioactive label or an enzyme for colorimetric detection or a colored colloidal particle such as gold, carbon, silica or latex for direct visualization of the immunoreaction (Leuvering JHW, Thal PJHM, Van der Waart M, Schuurs AHWM (1980) J Immunoassay 1(1): 77-91),.

In practice, colloidal gold particles or gold nanoclusters having a diameter of 25-40 nm are probably the most commonly applied labels in lateral flow immunoassays (Verheijen, R., in: Analytical Biotechnology 2002, Birkhauser Verlag Ed: T. Schalkhammer p134-166).

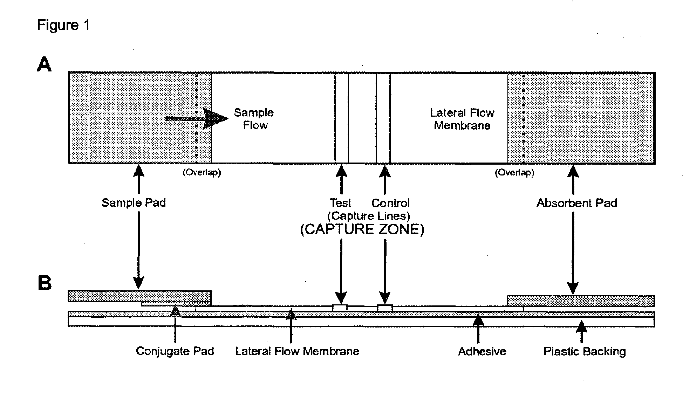

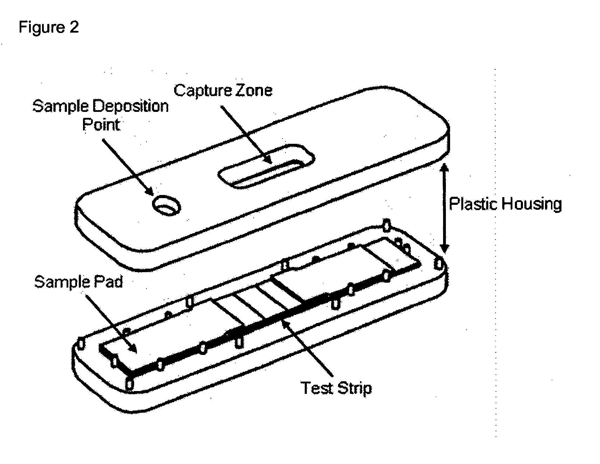

A typical immunoassay comprises a test strip such as the one exemplified in figure 1. Such a test strip may be mounted in a plastic housing such as the one exemplified in figure 2.

In a commercial setting, a typical test strip may be made up of a number of components, including a sample pad, a conjugate pad, an absorbent pad and a lateral flow membrane that contains the capture reagents at the capture zone, usually in the form of two capture lines; a test line and a control line. The capture reagent is typically immobilized on the lateral flow membrane. The capture reagent in the test comprises a molecule that is capable of binding to the analyte (capturing it). This molecule is herein referred to as a capture molecule.

The main purpose of the housing is to fixate the several components of the test strip and to keep them in close contact with each other. Moreover, the housing may determine the dimensions of the sample well and usually contains the viewing window with readout indications.

When describing the principle of the immunoassay, one has to distinguish between the direct assay that is usually used to detect high molecular mass analytes such as proteins, and the indirect or competitive assay usually used to detect low molecular mass analytes such as drug residues, antibiotics, hormones, etc.

In both types of tests, the user dispenses a liquid sample (buffer extract, milk, urine, serum, plasma, whole blood, etc) on to the sample pad. In a typical immunoassay, the sample then flows through the sample pad into the conjugate pad, where it releases and mixes with the conjugate. Other configurations are also known in the art, for instance where the sample is first contacted with the capture reagents and the conjugate is then subsequently contacted with the capture region (Verheijen, R., in: Analytical Biotechnology 2002, Birkhauser Verlag Ed: T. Schalkhammer p134-166).

Currently available immunoassays often employ conjugates that are located adjacent or downstream from the sample deposition point (figure 1). The sample itself then is relied upon to re-suspend the conjugate from the conjugate pad and carry it to the capture zone. Such immunoassays may require a relatively large sample size in order to provide an adequate flow of the sample and the conjugate.

Alternatively, the conjugate may be located upstream from the sample deposition point. Such immunoassays require a much smaller sample volume. Additional diluent can then be added to resuspend the conjugate from the conjugate pad and to provide an adequate flow of the sample and the conjugate to the capture zone.

Note that "upstream" and "downstream" refer to the position of an item relative to the direction of flow of a sample in the immunoassay.

In the direct assay format for detecting high molecular mass analytes such as proteins, the conjugate may consist of gold nanoclusters coated with specific antibodies reactive with the analyte. If that analyte is present in the sample it will react with the conjugate. The formed analyte--conjugate complexes are mobile and able to move freely from the conjugate pad into the membrane with the flow of the fluid. At the test line, the complexes may be captured by the capture molecule, such as immobilized anti-analyte antibodies. Thereby, the presence of the analyte in the sample will result in a colored test line. The color intensity of the test line may be proportional to the concentration of the analyte in the sample.

When the concentration of the analyte is lower than the lowest detection concentration or when the analyte is completely absent, no test line will be visible. Excess sample that flows beyond the test and control lines is taken up in the absorbent pad (Verheijen, R., in: Analytical Biotechnology 2002, Birkhauser Verlag Ed: T. Schalkhammer, p134-166).

In the competitive assay format for detecting low molecular mass analytes, the conjugate consists of gold nanoclusters coated with antibodies to the analyte. The test line here consists of the analyte coupled to a so-called carrier protein. The more analyte present in the sample, the more effectively it will compete with the immobilised analyte on the membrane for binding to the limited amount of antibodies of the conjugate. Thus, the absence of the analyte in the sample will result in a colored test line, whereas an increase in the amount of analyte will result in a decrease of signal in the detection zone. At a certain concentration of analyte in the sample, the test line will be no longer visible. The limit of detection is defined as the amount of analyte in the sample that just causes total invisiblity of the test capture line.

Immunoassays comprising a strip test are commercially available for an increasing number of analytes (high- and low-molecular mass). The first major target analyte for this test format was human chorionic gonadotropin (HCG) for the detection of pregnancy. At present, a great variety of immunoasays are available (Anonymous, Syllabus of a two-day seminar on Solid Phase Membrane-Based Immunoassays, Paris, September 25-26th, 1997, Millipore Corporation, Bedford, MA, USA, Anonymous, Syllabus of The Latex Course, London, October 1-3th, 1997, Organised by Bangs Laboratories, Inc., Fishers, IN, USA, Price CP, Thorpe GHG, Hall J, Bunce RA (1997) In: CP Price, DJ Newman (eds): Principles and Practices of Immunoassays (2nd edition). MacMillan Reference Ltd, London, 579-603, Hobbs FDR, Delaney BC, Fitzmaurice DA et al (1997). Health Technology Assessment 1(5)).

Examples of commercially available immunoassays are e.g. immunoassays for the detection of hormones (pregnancy, fertility, ovulation, menopause, sexual disorder and thyroid functions), tumour markers (prostate, colorectal, etc.), viruses (HIV, Hepatitis B and C), bacteria (Streptococcus A and B, Chlamydia trachomatis, Treponema pallidum, Heliobacter pylori, etc.), IgE (allergy) and troponin T in cardiac monitoring. All these analytes are measured on the basis of their presence or absence. An extensive review of near patient testing in primary care has been published by Hobbs et al., vide supra.

Although immunoassays appear simple, complex interactions among their various components lead to a number of challenges in both the development and manufacturing environments. These challenges are even greater for quantitative tests where the color intensity of the test line must be repeatable between production lots. From a developmental perspective, creating a successful test system means optimizing the interactions between its raw materials, component design, and manufacuring techniques.

In a typical immunoassay it is distinguished between a mobile phase and an immobile phase. The mobile phase consists of all molecules that may move over the membrane and/or the different pads used in the immunoassay, whereas the immobile phase consists of those molecules that do not move over the pads and or the membrane. Typically, the mobile phase comprises the sample, the analyte in the sample, the diluents and the conjugate, whereas the immobile phase typically comprises the capture reagents, immobilized in the control capture lines and the test capture lines.

A typical dilemma common to every immunoassay is that it is desired that the mobile phase can freely move over or through the membrane while experiencing as less hindrance as possible from the membrane whereas the immobile phase should be irremovably attached to the membrane. The pore size of the membrane determines the flow rates of the mobile phase. For fast assays, a fast flowing membrane is preferred and such membranes are characterized by relatively large pore sizes. It is a problem for such membranes to retain the capture reagents in the immobile phase, in particular if the capture reagent consists of small molecules, such as peptides.

Nitrocellulose is probably the most commonly used polymer for lateral flow membranes in immunoassay. The pore size of such membranes varies between 5 to 20 µm, which is large compared to a pore size of 0.2 to 1.2 µm of a nitrocellulose membrane used for protein blotting. A large pore size implies a small membrane surface area and consequently a low protein binding capacity, i.e. 20-30 µg IgG/cm² instead of the 110 µg IgG/cm² for a blotting membrane with a pore size of 0.45 µm.

Many immunoassays have been described in the art and methods for the immobilization of capture reagents, most notably antigens or antibodies, are known in the art. In general, methods for immobilizing small peptides employ the principle of conjugating the small peptide to a larger molecule such as for instance a larger peptide, a protein such as bovine serum albumine (BSA) or a sugar. It has also been described to use fusion proteins, commonly produced in recombinant organisms.

A method well-known in the art is coupling of a capture molecule such as a small peptide to larger proteins such as BSA or ovalbumin in order to obtain a capture reaqent that is better retained on a membrane than the capture molecule itself. (Verheijen, R., in: Analytical Biotechnology 2002, Birkhauser Verlag Ed: T. Schalkhammer, p134-166). Other known methods are based on the immobilization of a capture molecule to a solid support wherein the high affinity binding of biotin and streptavidin is employed. In those prior art methods, avidin or streptavidin is first immobilized on a solid support and thereafter used to capture a biotinylated capture molecule such as a peptide.

The coupling of biotin to other molecules is a process known in the art as biotinylation. Biotin binds strongly to proteins like avidin and streptavidin, and biotionylated molecules are easily captured by a surface having avidin or streptavidin molecules immobilized thereon. The biotin-avidin and biotin-streptavidin complexes have extremely large association constants (Kₐ =10¹⁵ M⁻¹ for avidin and 10¹³ M⁻¹ for streptavidin), energetically equivalent to covalent bonds, and are stable over a wide range of temperature and pH.

Biotinylation is typically accomplished using a chemically active form of biotin to label exposed lysine residues on target proteins. Lysine is one of the most frequently occurring amino acids, and chemical biotinylation can, therefore, be used to biotinylate essentially all proteins.

Biotin/streptavidin interactions have been utilized in other ways in immunoassay procedures for some time. For example, in U.S. 5,126,241, streptavidin adsorbed to a solid support is used to bind biotinylated antigen in a procedure which involves incubation to form a complex in which the analyte to be determined competes with label and solid support for access to an antibody capable of binding all three.

U.S. 4,496,654 describes an assay for human chorionic gonadotropin conducted by capturing a biotinylated antibody using an avidin-coupled paper disk, reacting the antibody on a disk with a solution suspected of containing hCG, and then determining the amount of hCG on the disk using standard determination techniques. This assay results in a sandwich of hCG formed from anti- hCG bound to solid support through biotin/avidin linkage and labeled anti-hCG. This assay does not involve a lateral flow of sample.

U.S. 5,001,049 describes a method for determining antibodies against human HIV which involves incubating streptavidin-derivatized solid support with a biotinylated peptide reactive with anti-HIV, and then detecting any bound antibody with labeled antibody receptor. Again, lateral flow does not take place in these assays.

RE 34,132, which is a reissue of Patent Number 4,945,042, describes a direct assay for an antibody wherein the analyte is an antibody that serves as a link between a labeled epitope and an epitope bound to substrate through a streptavidin/biotin link. Again, speed of reaction is not critical, since a lateral flow format is not required.

It is the subject of this application to provide at least an alternative technology for the immobilization of capture reagents in immunoassays, preferably an immobilization technology with advantages over the prior art. Such advantages may be found in the specificity, sensitivity, costs or ease of manufacturing of the immunoassays and or in advantages in costs or ease of handling of the eventual diagnostic assay itself.

### Summary of the invention

Surprisingly, it was found that an improved specificity and or sensitivity could be achieved when a capture molecule such as an antigen or antibody was immobilized on a membrane while conjugated to biotin and a binding member such as an anti-biotin antibody, avidin, captavidine, streptavidin, neutravidin or streptavidin-hydrazide or derivatives thereof before immobilizing the thus obtained complex on the solid support.

The invention therefore relates to a method for the immobilization of capture molecule on a solid support wherein the capture molecule is covalently attached to a biotin molecule to obtain a biotinylated capture molecule and wherein the biotinylated capture molecule is subsequently contacted with a binding member to form a complex where after the complex is contacted with the solid support.

### Detailed description of the invention

It was found that a method wherein a capture molecule was labeled with biotin and then conjugated to a binding member such as an anti-biotin antibody, streptavidin, avidin, neutravidin or streptavidin-hydrazide in solution before immobilizing the thus obtained complex to a solid support, yielded particular advantages over the known immobilization techniques wherein the biotin labeled molecule is captured by a binding member already immobilized on the solid support.

The invention therefore relates to a method for the immobilization of a capture molecule on a solid support wherein the capture molecule is covalently attached to a biotin molecule to obtain a biotinylated capture molecule and wherein the biotinylated capture molecule is first contacted with a binding member to form a complex whereafter the complex is contacted with the solide support.

In the prior art methods, the coupling of biotin to a binding member is typically performed while the binding member is attached to a solid support. This invention is directed to the immobilization of the capture molecule after a complex is formed between the biotinylated capture molecule and a binding member in solution. Only thereafter the complex comprising the biotinylated capture molecule and the binding member is then immobilized on the solid support.

A binding member in this context is to be interpreted as a molecule capable of binding to biotin. This may be a molecule capable of forming a reversible binding with biotin such as for instance an anti-biotin antibody. It may also be a high affinity binding member such as a molecule with a higher affinity constant. Such a high affinity binding member may form a bond with the biotinylated complex that is comparable to a covalent bond. The skilled person will recognize this to be in the order of Kₐ =10¹⁵ M⁻¹ to 10¹¹ M⁻¹, typically 10¹³ M⁻¹ for streptavidin.

The high affinity binding member may advantageously be selected from the group consisting of avidin, captavidine, streptavidin, neutravidin or streptavidin-hydrazide or derivatives thereof.

It is to be understood that this method differs from the prior art methods described above in that the binding member is not coupled to the solid support when mixed with the biotinylated capture molecule; only after the formation of the bond between biotin and the binding member, the thus obtained complex is immobilized on the solid support. For the avoidance of doubt, the contacting of the biotinylated capture molecule with a binding member to form a capture molecule complex is performed in solution or in any other form wherein neither the biotinylated capture molecule nor the binding member is attached to a solid support.

The term capture molecule in this context is to be interpreted as a molecule capable of binding to another molecule such as an analyte, in particular an antigen or antibody. Antigens may consist of a wide range of molecules such as haptens, sugars, peptides, proteins oligosaccharides and many more known by the skilled person. Antibodies may be monoclonal or polyclonal antibodies. Antibodies may be derived from human clinical samples or bodily fluids such as for instance saliva, sweat, sputum, urine, blood, plasma, serum, vaginal fluid or cerebrospinal fluid. They may also be derived from experimental animals, such as mice rats, rabbits, lama's, and camels or from recombinant sources such as bacteria, yeasts or human cells.

The method according to the invention may advantageously be employed when the capture molecule consists of small molecules. In this context, the term small molecule is to be interpreted as meaning a molecule with a molecular mass below 100,000 (hundred thousand) Da, preferably below 50,000 Da, such as 40,000, 30,000, 20,000 or 10,000 Da. Particularly advantages are obtained when the small molecule is even smaller than 10,000 Da, such as for instance smaller than 8000, 6000, 4000, 2000 or 1000 Da. Examples of such small molecules may be antibody fragments, synthetic of natural peptides or haptens.

Alternatively, the term small molecules may also be functionally defined, for instance as a function of the pore size of the solid support such as a membrane. It is known in the art that the adhesion or binding of molecules to solid supports depends on the pore size of the solid support. If the pore size is bigger, binding of a capture molecule such as a peptide to the solid support will be poorer than the binding to a solid support with smaller pores. Thus, a small molecule may also be defined as a molecule that will not or not detectably stick to a given membrane with a particular pore size.

Particularly advantageous results were obtained when the small molecules were peptides. In this study, peptides were used in the range of 5 to 100 amino acids, such as for instance 10, 12, 14, 16, 19, 20, 30, 50 or 80 amino acids. Such peptides are known for their poor binding to solid supports, in particular porous membranes, more in particular porous membranes with large pore sizes. When such peptides were coupled to biotin and used in the method according to the invention, improved sensitivity and specificity of the resulting assay was observed. Moreover, the method of manufacturing was more easy and less labor intensive as compared with the prior art methods. In addition, the resulting lines of positive samples at the detection region were sharper and clearer when compared to prior art methods.

The term solid support in this respect is to be interpreted as any support or surface suitable for performing an immunoassay. In particular the method employs a solid support that is porous to allow a capillary flow through the material. Examples of such porous solid supports are nitrocellulose membranes.

Methods for covalently attaching a biotin molecule to any capture molecule such as a small molecule are known in the art, and kits for biotinylation are commercially available, for instance from Roche Diagnostics, Indianapolis, Cat No. 11 008960 001. The skilled person will know how to handle and bind a biotinylated capture molecule with a binding member or a high affinity binding member such as avidin, captavidine, streptavidin, neutravidin or streptavidin-hydrazide or derivatives thereof to the biotinylated capture molecule.

It was tried to bind synthetic peptides directly to nitrocellulose membranes Millipore HF075, HF090, HF120, HF135, HF180 or HF240. For that purpose, the following synthetic 12-mer peptides as described in WO 03/050542 were spotted on the membranes and dried.

| | |
|---|---|
| 0002-27 | H Q K R G Cit G W S R A A |
| 0002-29 | H Q R R V Cit G W S R A A |
| 0002-31 | H Q R R T Cit G G S R A A |
| 0002-32 | H Q R K W Cit G A S R A A |
| 0002-36 | H Q F R F Cit G Cit S R A A |
| 0002-37 | H Q K W R Cit G R S Cit A A |
| 0002-63 | H Q F R F Cit G W S R A A |
| 0107-32 | K P Y T V Cit K F M R R P |
| 0107-35 | A R F Q M Cit H Cit R L I R |
| 0107-45 | Y S F V W Cit S H A R P R |
| 0113-30 | A R F Q M R H Cit R L I R |
| 0218-36 | R N L R L Cit R E R N H A |

Herein "Cit" depicts citrulline and the other amino acids are shown in the one-letter code for amino acids. In addition, the cyclic variants of these peptides (example 1) were also tested in the same setting.

These linear and cyclic peptides are known for their excellent reactivity with antibodies in the sera from patients suffering from Rheumatoid Arthritis. The dried membranes were tested for reactivity with 20 of such sera ranging from low to very high titers of antibody and it appeared that none of the peptides were retained on the membranes, which was evident by the fact that none of the lines where the peptides were applied gave a significant reaction with the antibodies.

The same peptides were then biotinylated (example 2) and tested for reactivity in the same manner as described above. Again, no reaction was observed, which was attributed to the fact that the biotinylated peptides were again too small to be retained by the membranes. In the examples section the results are shown obtained with the reference panel of high, medium and low plasma samples only.

In order to provide for a comparative example, a prior art method was used to immobilize the peptides more effectively. Therefore, a conjugate was prepared between the peptides and bovine serum albumin (BSA) and that conjugate was spotted onto nitrocellulose. Some false positive results were obtained as well as false negatives. In total this was rated as an average result (Table 1).

Next, the biotinylated peptides were reacted with a binding member comprising anti-biotin monoclonal or polyclonal antibodies (Jacksons laboratories, product 200-002-096 IgG fraction) and then spotted onto the membranes. Although this improved the sensitivity of the assay, the same specificity was observed as with the prior art method employing a peptide-BSA conjugate (Tables 2 and 3).

When the biotinylated peptides were mixed with a high affinity binding member such as avidin, captavidine, streptavidin, neutravidin or streptavidin-hydrazide or derivatives thereof before immobilizing the capture molecule complex on the membranes, the results greatly improved. Less false positives were observed in comparison to any of the above methods and good specificity was obtained. In particular streptavidin worked very well, this resulted in an assay with 100% specificity and 100% sensitivity (Tables 2 and 3).

In the examples, several peptides were tested for their reactivity with antibodies from patient with Rheumatoid Arthritis (RA). It was shown that in all cases an improved reactivity was observed in comparison to prior art methods. Improved reactivity in this sense is to be interpreted as an improved sensitivity or specificity of the assay or both. The invention therefore also relates to a method as described above wherein the capture molecule is an antigen reactive with antibodies obtained from patients suffering from Rheumatoid Arthritis.

Improved reactivity with RA antibodies was observed when a cyclic citrullinated peptide was used as the capture molecule (Table 3).

The method according to the invention may be employed with a wide range of solid supports. Advantageous results were obtained when the solid support was selected from the group consisting of fast flowing membranes such as Millipore HF075, HF090, HF120, HF135, HF180 or HF240 or membranes with comparable flow properties.

The invention also relates to a solid support with a capture molecule complex immobilized thereon, obtainable by the process according to the invention. Such a solid support may be used for instance in an immunoassay for the detection of antibodies or antigens, such as detection of antibodies specific for Rheumatoid Arthritis. In such an immunoassay a cyclic citrullinated peptide is advantageously immobilized on the solid support.

In the examples it is described how several capture molecule complexes were contacted with the solid support. This was done with the help of a machine which is not to be interpreted as that this is mandatory. A skilled person will be aware of other options for depositing a capture molecule complex onto a solid support, including but not limited to dipping, spraying, blotting, spotting and many others.

### Legends to the figures

Figure 1. Schematic of a typical test strip. A. Top view, B. Side view.
Figure 2: Example of a housing used in a strip test device.

### Examples

### Example 1: Peptides

The following linear peptides were obtained commercially from Polypeptide Laboratories, 365 Maple Avenue, Torrance CA 90503.

| | |
|---|---|
| 2-27 | H Q K R G Cit G W S R A A |
| 2-29 | H Q R R V Cit G W S R A A |
| 2-31 | H Q R R T Cit G G S R A A |
| 2-32 | H Q R K W Cit G A S R A A |
| 2-36 | H Q F R F Cit G Cit S R A A |
| 2-37 | H Q K W R Cit G R S Cit A A |
| 2-63 | H Q F R F Cit G W S R A A |
| 107-32 | K P Y T V Cit K F M R R P |
| 107-35 | A R F Q M Cit H Cit R L I R |
| 107-45 | Y S F V W Cit S H A R P R |
| 113-30 | A R F Q M R H Cit R L I R |
| 218-36 | R N L R L Cit R E R N H A |

Cyclic variants of these peptides were also obtained commercially from Polypeptide Laboratories, 365 Maple Avenue, Torrance CA 90503. These peptides were cyclisized through their cysteine residues and had the following primary sequence:

| | |
|---|---|
| Cyclic 2-27 | G S Q H C **H Q K R G Cit G W S R A A** C G-NH2 |
| Cyclic 2-29 | G S Q H C **H Q R R V Cit G W S R A A** C G-NH2 |
| Cyclic 2-31 | G S Q H C **H Q R R T Cit G G S R A A** C G-NH2 |
| Cyclic 2-32 | G S Q H C **H Q R K W Cit G A S R A A** C G-NH2 |
| Cyclic 2-36 | G S Q H C **H Q F R F Cit G Cit S R A A** C G-NH2 |
| Cyclic 2-37 | G S Q H C **H Q K W R Cit G R S Cit A A** C G-NH2 |
| Cyclic 2-63 | G S Q H C **H Q F R F Cit G W S R A A** C G-NH2 |
| Cyclic 107-32 | G S Q H C **K P Y T V Cit K F M R R P** C G-NH2 |
| Cyclic 107-35 | G S Q H C **A R F Q M Cit H Cit R L I R** C G-NH2 |
| Cyclic 107-45 | G S Q H C **Y S F V W Cit S H A R P R** C G-NH2 |
| Cyclic 113-30 | G S Q H C **A R F Q M R H Cit R L I R** C G-NH2 |
| Cyclic 218-36 | G S Q H C **R N L R L Cit R E R N H A** C G-NH2 |
| | The C-terminus of the peptides was amidated. |

### Prior art example: Coupling of peptides to BSA

BSA-peptide complexes were prepared by cross-linking BSA to the linear and cyclic peptides as shown in example 1. For that purpose, the peptides were synthesized wherein a cysteine residue was added to the N-terminal side of the peptides shown in example 1. Cross-linking of BSA was achieved using the cross-linker sulfo-SMCC obtained from Pierce, Meridian Road Rockford IL, product nr 22322 according to the manufacturer's protocol. In essence: BSA was activated when dissolved at 1mg/ml in PBS and 20 ug crosslinker was added per mg BSA. The mixture was incubated at room temperature for 1 hour and the reaction was blocked with 1 mM Tris-HCl, pH 7.4 final concentration. Free cross-linker was removed with a PD10 column. Peptides were added at 1 mg per mg of activated BSA and incubated for 2 hours at room temperature. Free peptides were removed by dialysis against PBS pH 7.4.

Polyacrylamide gel analysis revealed a molecular weight of 100kDa for the BSA-peptide complex, meaning that on average about 13 peptide molecules were attached to one BSA molecule.

The following peptides were obtained.

| | |
|---|---|
| BSA 2-27 | BSA C H Q K R G Cit G W S R A A |
| BSA 2-29 | BSA C H Q R R V Cit G W S R A A |
| BSA 2-31 | BSA C H Q R R T Cit G G S R A A |
| BSA 2-32 | BSA C H Q R K W Cit G A S R A A |
| BSA 2-36 | BSA C H Q F R F Cit G Cit S R A A |
| BSA 2-37 | BSA C H Q K W R Cit G R S Cit A A |
| BSA 2-63 | BSA C H Q F R F Cit G W S R A A |
| BSA 107-32 | BSA C K P Y T V Cit K F M R R P |
| BSA 107-35 | BSA C A R F Q M Cit H Cit R L I R |
| BSA 107-45 | BSA C Y S FV W Cit S H A R P R |
| BSA 113-30 | BSA C A R F Q M R H Cit R L I R |
| BSA 218-36 | BSA C R N L R L Cit R E R N H A |

### Example 2: Biotinylation of capture molecules

The set of linear and cyclic peptides shown in Example 1 were also obtained Polypeptide Laboratories, 365 Maple Avenue, Torrance CA 90503 with an N-terminal biotin residue. These peptides had the following primary structure:

| | |
|---|---|
| Biotin 2-27 | Biotin H Q K R G Cit G W S R A A |
| Biotin 2-29 | Biotin H Q R R V Cit G W S R A A |
| Biotin 2-31 | Biotin H Q R R T Cit G G S R A A |
| Biotin 2-32 | Biotin H Q R K W Cit G A S R A A |
| Biotin 2-36 | Biotin H Q F R F Cit G Cit S R A A |
| Biotin 2-37 | Biotin H Q K W R Cit G R S Cit A A |
| Biotin 2-63 | Biotin H Q F R F Cit G W S R A A |
| Biotin 107-32 | Biotin K P Y T V Cit K F M R R P |
| Biotin 107-35 | Biotin A R F Q M Cit H Cit R L I R |
| Biotin 107-45 | Biotin Y S F V W Cit S H A R P R |
| Biotin 113-30 | Biotin A R F Q M R H Cit R L I R |
| Biotin 218-36 | Biotin R N L R L Cit R E R N H A |

### Biotinylated and Cyclic:

| | |
|---|---|
| Biotin-Cyclic 2-27 | Biotin G S Q H C **H Q K R G Cit G W S R A A** C G-NH2 |
| Biotin-Cyclic 2-29 | Biotin G S Q H C **H Q R R V Cit G W S R A A** C G-NH2 |
| Biotin-Cyclic 2-31 | Biotin G S Q H C **H Q R R T Cit G G S R A A** C G-NH2 |
| Biotin-Cyclic 2-32 | Biotin G S Q H C **H Q R K W Cit G A S R A A** C G-NH2 |
| Biotin-Cyclic 2-36 | Biotin G S Q H C **H Q F R F Cit G Cit S R A A** C G-NH2 |
| Biotin-Cyclic 2-37 | Biotin G S Q H C **H Q K W R Cit G R S Cit A A** C G-NH2 |
| Biotin-Cyclic 2-63 | Biotin G S Q H C **H Q F R F Cit G W S R A A** C G-NH2 |
| Biotin-Cyclic 107-32 | Biotin G S Q H C **K P Y T V Cit K F M R R P** C G-NH2 |
| Biotin-Cyclic 107-35 | Biotin G S Q H C **A R F Q M Cit H Cit R L I R** C G-NH2 |
| Biotin-Cyclic 107-45 | Biotin G S Q H C **Y S F V W Cit S H A R P R** C G-NH2 |
| Biotin-Cyclic 113-30 | Biotin G S Q H C **A R F Q M R H Cit R L I R** C G-NH2 |
| Biotin-Cyclic 218-36 | Biotin G S Q H C **R N L R L Cit R E R N H A** C G-NH2 |

### Example 3: Preparation of capture molecule complexes using avidin, streptavidin, neutravidin or streptavidin-hydrazide.

Peptides as described above were dissolved in Phosphate Buffered Saline (PBS) at a concentration of 1 mg/ml. They were mixed with a 1mg/ml solutions of either avidin, streptavidin, neutravidin or streptavidin-hydrazide in PBS in a molar ratio of peptide : avidin, streptavidin, neutravidin or streptavidin-hydrazide of 5 : 2. The mixture was incubated at room temperature for 10 minutes and used immediately. Avidin, streptavidin, neutravidin and streptavidin-hydrazide were obtained from Pierce.

In a comparative experiment wherein the peptides were mixed with avidin, streptavidin, neutravidin or streptavidin-hydrazide in molar ratios of 1:1, 2:1, 4:1 and 5:2 and tested with a limited set of RA antibodies. The 5:2 molar ratio appeared to be optimal. The other ratios still yielded acceptable results, it is therefore concluded that the ratio is not critical and can be optimized for each individual capture molecule complex. In general it will be advantageous to use a molar excess of peptide or protein.

### Example 4: Preparation of capture molecule complexes using monoclonal anti-biotin antibodies.

Biotinylated peptides as described above were dissolved in Phosphate Buffered Saline (PBS) at a concentration of 1 mg/ml. They were mixed with a 1.3 mg/ml solution of monoclonal anti-biotin antibody obtained from Jacksons laboratories, product 200-002-096 IgG fraction. The mixture was incubated at 37 °C for 30 minutes and used immediately.

### Example 5: immobilization of capture molecule complexes on a solid support.

Capture molecule complexes were spotted in the form of a thin line on a solid support using a Kinematic 1600 machine. Solid supports used were Millipore HF075, HF090, HF120, HF135, HF180 and HF240 membranes. The stripe rate was 0.9 ul/cm at a speed of 10 cm/sec. Membranes were blocked using a PBS solution containing 1% Bovine Serum Albumin (BSA) and 1% Pluronic F68, a commercially available surfactant. For that purpose, the entire strip was sprayed using a Biodot XYZ 3000 machine equipped with an air jet dispensing head using the following settings: bed speed 5 cm/sec, dispenser 10 ul/sec, y axis 20 and micrometer 0,0. The membranes were dried in a dry room at room temperature (21 °C) for 24 hours and cut into strips of 5mm width. The length of the strips was about 30 mm and the capture molecule complexes were striped at about 7 mm from the top. The results obtained with the various membrane types were essentially the same. HF 180 membranes provided an optimal mix of flow properties and sensitivity/specificity. Those results are shown in tables 1 - 3, the results obtained with the other membranes were comparable if not identical.

### Example 6: Performing an immunoassay, i.e. a lateral flow assay

An absorbent pad was attached to the top of the strip and the bottom of the strip was placed in an Eppendorf reaction vessel of 1.5 ml containing 75 ul of antibody solution. The antibody solution was allowed to fully migrate into the strip. The strip was then placed in another vessel containing 75 ul of a conjugate solution and a fluid stream was allowed to reach the absorbent pad. This was allowed to carry on for 20 minutes at room temperature.

Antibody solutions were prepared by mixing 15 ul human plasma with 60 ul PBS/ 1% BSA. Four different human plasma samples were used:
a) Normal Human plasma, non-reactive
b) Low titer Rheumatoid Arthritis plasma
c) Medium titer Rheumatoid Arthritis plasma
d) High titer Rheumatoid Arthritis plasma.

The plasma samples were derived from a master batch obtained from Trina International Nanikon in Switzerland. Cat. Nr DA 1708. High titer plasma was prepared by diluting the master batch plasma in normal human plasma until a titer of 1600 Units/ml was obtained when measured in the EuroDiagnostica Immunoscan RA anti CCP Test Kit; EuroDiagnostica BV Arnhem, The Netherlands, Cat No RA-96RT. Medium titer plasma was obtained by diluting the High titer plasma until a titer of 400 Units/ml was obtained, the low titer plasma was obtained by further dilution in normal human plasma until a titer of 25 Units/ml was obtained.

Conjugate solution was prepared by mixing 30 ul of a gold conjugate obtained from British Biocell International (BBI); anti-human IgG gold conjugate Cat. No: BA.GAHL 40 with 70 ul running buffer. Running buffer used consisted of PBS with 1% BSA.

The color intensity of the line on each device was determined using a visual scale ("Rann scale") ranging from 0-11, wherein 0 represents no color and 11 represents the most intense color. The RANN scale is used as a comparator, where the test signal intensity is compared to the equivalent RANN scales intensity and assigned a value for result recording purposes. The RANN score card consists of 5 lines with varying intensity ranging from very faint to very intense. The extremes on the card correspond to line intensities of 1 and 10. If the intensity of a test line is below the least intensive line on the score card the intensity of the test line is scored as 0. If the intensity of a test line equals the least intensive line on the score card, the intensity of the test line is scored as 1. If the intensity of a test line is in between the least intensive line and the next intensive line on the score card, the intensity of the test line is scored as 2 and so on. Maximum score obtainable was 11 meaning more intense than the most intense line on the RANN score card.

The intensity the stripes in each test zone that was obtained with the materials as described above is summarized in Tables 1 - 3.

**Table 1: RANN Score of test lines obtained with the various capture molecules and capture molecule complexes according to the prior art.**

| **Peptide** | **linear** | **cyclic** | **Biotin Linear** | **Biotin Cyclic** | **BSA Linear** | **BSA Cyclic** |
|---|---|---|---|---|---|---|
| 2-27 | 0/0/0/0 | 0/0/0/0 | 0/0/0/0 | 0/0/0/1 | 0/0/1/4 | 1/0/0/4 |
| 2-29 | 0/0/0/0 | 0/0/0/0 | 0/0/0/0 | 0/0/0/0 | 0/1/2/4 | 2/4/4/4 |
| 2-31 | 0/0/0/0 | 0/0/0/0 | 0/0/0/0 | 0/0/0/0 | 3/3/3/4 | 2/2/5/5 |
| 2-32 | 0/0/0/1 | 0/0/0/0 | 0/0/0/0 | 0/0/0/0 | 0/0/0/0 | 2/2/2/4 |
| 2-36 | 0/0/0/0 | 0/0/0/0 | 0/0/0/0 | 0/0/0/0 | 0/0/2/3 | 0/0/3/3 |
| 2-37 | 0/0/0/0 | 0/0/0/1 | 0/0/0/0 | 0/0/0/0 | 2/2/3/3 | 1/0/0/0 |
| 2-63 | 0/0/0/0 | 0/0/0/0 | 0/0/0/0 | 0/0/0/0 | 0/0/0/2 | 0/0/2/4 |
| 107-32 | 0/0/0/0 | 0/0/0/0 | 0/0/0/1 | 1/0/0/0 | 0/0/0/0 | 0/0/0/3 |
| 107-35 | 0/0/0/0 | 0/0/0/0 | 0/0/0/0 | 0/0/0/0 | 0/0/0/4 | 0/0/0/0 |
| 107-45 | 0/0/0/0 | 2/0/0/0 | 0/0/0/0 | 0/0/0/0 | 2/0/0/3 | 1/0/0/0 |
| 113-30 | 0/0/0/0 | 0/0/0/0 | 0/0/0/1 | 0/0/0/0 | 0/0/0/3 | 0/0/2/4 |
| 218-36 | 0/0/0/0 | 0/0/0/0 | 1/0/0/0 | 0/0/0/0 | 1/0/0/0 | 0/0/0/0 |

| | | | | | | |
|---|---|---|---|---|---|---|
| Table 1: RANN score of normal human serum/low titer/medium titer/high titer plasma samples obtained with test strips carrying the capture molecules and capture molecule complexes as indicated. | | | | | | |

**Table 2: RANN Score of test lines obtained with the various capture molecules and capture molecule complexes according to the invention in comparison to the prior art linear BSA conjugates. Results with Linear Peptides are shown**

| **Peptide** | **BSA Linear** | **Avidin-biotin-peptide** | **Streptavidin-biotin-peptide** | **Neutravidin-biotin-peptide** | **Streptavidin-hydrazide-biotin-peptide** | **Antibody-biotin-peptide** |
|---|---|---|---|---|---|---|
| 2-27 | 0/0/1/4 | 0/2/4/4 | 0/2/4/6 | 0/2/4/6 | 0/2/4/4 | 1/0/1/4 |
| 2-29 | 0/1/2/4 | 0/2/4/4 | 0/1/4/6 | 0/2/4/4 | 0/2/4/4 | 2/4/4/4 |
| 2-31 | 3/3/3/4 | 0/2/3/3 | 0/2/4/6 | 0/2/3/5 | 2/2/2/3 | 2/2/5/5 |
| 2-32 | 0/0/0/0 | 0/2/3/3 | 0/2/3/6 | 0/2/3/4 | 0/2/2/3 | 2/2/2/4 |
| 2-36 | 0/0/2/3 | 0/2/3/3 | 0/2/4/6 | 0/2/3/6 | 0/1/3/3 | 0/0/3/3 |
| 2-37 | 2/2/3/3 | 1/2/4/6 | 0/2/3/6 | 0/2/4/6 | 1/2/5/6 | 1/0/1/0 |
| 2-63 | 0/0/0/2 | 0/2/4/6 | 0/3/4/6 | 0/2/4/6 | 0/2/2/6 | 4/0/2/4 |
| 107-32 | 0/0/0/0 | 0/2/2/4 | 0/1/2/7 | 0/2/2/2 | 0/2/2/2 | 1/0/0/3 |
| 107-35 | 0/0/0/4 | 1/2/2/3 | 0/1/2/4 | 0/2/2/3 | 2/2/2/3 | 0/0/0/0 |
| 107-45 | 2/0/0/3 | 0/2/3/6 | 0/1/2/4 | 0/2/3/6 | 0/2/3/6 | 1/0/0/0 |
| 113-30 | 0/0/0/3 | 1/2/4/4 | 0/1/1/4 | 1/2/4/4 | 1/2/4/4 | 4/0/2/4 |
| 218-36 | 1/0/0/0 | 1/2/4/6 | 0/1/2/2 | 1/2/4/6 | 1/2/4/6 | 0/0/0/0 |

| | | | | | | |
|---|---|---|---|---|---|---|
| Table 2: RANN score of normal human serum/low titer/medium titer/high titer plasma samples obtained with test strips carrying the capture molecules and capture molecule complexes according to the invention as indicated. | | | | | | |

**Table 3: RANN Score of test lines obtained with the various capture molecules and capture molecule complexes according to the invention in comparison to the BSA conjugates. Results with Cyclic Peptides are shown.**

| **Peptide** | **BSA Cyclic** | **Avidin-biotin-peptide** | **Streptavidin-biotin-peptide** | **Neutravidin-biotin-peptide** | **Streptavidin-hydrazide-biotin-peptide** | **Antibody-biotin-peptide** |
|---|---|---|---|---|---|---|
| 2-27 | 1/0/0/4 | 0/2/4/7 | 0/2/6/11 | 0/2/4/8 | 1/2/6/8 | 1/0/0/8 |
| 2-29 | 2/4/4/4 | 0/2/4/7 | 0/1/6/10 | 0/2/4/8 | 0/2/6/6 | 3/4/4/8 |
| 2-31 | 2/2/5/5 | 0/0/5/6 | 0/2/6/9 | 0/2/3/7 | 0/2/6/8 | 2/2/5/8 |
| 2-32 | 2/2/2/4 | 0/2/5/6 | 0/2/6/9 | 0/2/3/6 | 1/2/5/7 | 3/2/2/4 |
| 2-36 | 0/0/3/3 | 0/2/5/8 | 0/2/5/9 | 1/2/3/7 | 0/1/7/7 | 3/0/3/3 |
| 2-37 | 1/0/0/0 | 0/2/5/9 | 0/2/5/9 | 0/2/4/8 | 1/2/5/8 | 1/0/8/8 |
| 2-63 | 0/0/2/4 | 0/2/5/9 | 0/2/7/11 | 0/2/4/9 | 0/2/2/8 | 4/0/8/6 |
| 107-32 | 0/0/0/3 | 0/2/2/6 | 0/1/4/9 | 0/2/2/9 | 0/2/8/8 | 1/2/6/6 |
| 107-35 | 0/0/0/0 | 1/2/2/7 | 0/1/4/9 | 0/2/2/9 | 1/2/3/5 | 0/2/2/7 |
| 107-45 | 1/0/0/0 | 0/0/3/7 | 0/1/2/8 | 0/2/3/9 | 0/2/3/8 | 1/3/4/6 |
| 113-30 | 0/0/2/4 | 0/2/4/8 | 0/1/3/8 | 0/2/4/6 | 1/2/4/8 | 4/0/2/4 |
| 218-36 | 0/0/0/0 | 1/2/4/7 | 0/1/4/8 | 0/2/4/6 | 1/2/4/8 | 0/2/2/6 |

| | | | | | | |
|---|---|---|---|---|---|---|
| Table 3: RANN score of normal human serum/low titer/medium titer/high titer plasma samples obtained with test strips carrying the capture molecules and capture molecule complexes according to the invention as indicated. | | | | | | |

## Claims

1. Method for the immobilization of a capture molecule on a solid support wherein the capture molecule is covalently attached to a biotin molecule to obtain a biotinylated capture molecule and wherein the biotinylated capture molecule is first contacted with a binding member to form a capture molecule complex where after the capture molecule complex is contacted with the solid support.

2. Method according to claim 1 wherein the binding member is a high affinity binder selected from the group consisting of avidin, captavidine, streptavidin, neutravidin or streptavidin-hydrazide or derivatives thereof.

3. Method according to claims 1 or 2 wherein the capture molecule is a small molecule.

4. Method according to claims 1 -3 wherein the capture molecule is a peptide.

5. Method according to claim 1 -4 wherein the capture molecule is an antigen reactive with antibodies obtained from patients suffering from Rheumatoid Arthritis.

6. Method according to claim 5 wherein the capture molecule is a cyclic citrullinated peptide.

7. Method according to claims 1 to 6 wherein the solid support is a nitrocellulose membrane.

8. Method according to claim 7 wherein the solid support is a fast flowing membrane.

9. Method according to claim 8 wherein the membrane is selected from the group consisting of Millipore HF075, HF090, HF120, HF135, HF180 or HF240 or membranes with comparable or equivalent flow properties.

10. A solid support with a capture molecule complex immobilized thereon, obtained by the method according to claims 1 to 9.

11. Immunoassay for the detection of antibodies or antigens comprising a solid support according to claim 10.

12. Immunoassay according to claim 11 for the detection of antibodies specific for Rheumatoid Arthritis.

13. Immunoassay according to claim 12 wherein a cyclic citrullinated peptide is immobilized on the solid support.

## Patentansprüche

1. Verfahren zur Immobilisierung eines Fängermoleküls auf einem festen Träger, wobei das Fängermolekül kovalent an ein Biotinmolekül unter Erhalt eines biotinylierten Fängermoleküls gebunden wird und wobei das biotinylierte Fängermolekül zunächst mit einem Bindungsglied unter Bildung eines Fängermolekülkomplexes in Kontakt gebracht wird, wonach der Fängermolekülkomplex mit dem festen Träger in Kontakt gebracht wird.

2. Verfahren nach Anspruch 1, wobei es sich bei dem Bindungsglied um ein aus der aus Avidin, Captavidin, Streptavidin, Neutravidin und Streptavidinhydrazid oder Derivaten davon bestehenden Gruppe ausgewähltes hochaffines Bindungsmittel handelt.

3. Verfahren nach Anspruch 1 oder 2, wobei es sich bei dem Fängermolekül um ein kleines Molekül handelt.

4. Verfahren nach Anspruch 1-3, wobei es sich bei dem Fängermolekül um ein Peptid handelt.

5. Verfahren nach Anspruch 1-4, wobei es sich bei dem Fängermolekül um ein Antigen handelt, das mit von an rheumatoider Arthritis leidenden Patienten erhaltenen Antikörpern reagiert.

6. Verfahren nach Anspruch 5, wobei es sich bei dem Fängermolekül um ein cyclisches citrulliniertes Peptid handelt.

7. Verfahren nach Anspruch 1 bis 6, wobei es sich bei dem festen Träger um eine Nitrocellulosemembran handelt.

8. Verfahren nach Anspruch 7, wobei es sich bei dem festen Träger um eine Fast-Flow-Membran handelt.

9. Verfahren nach Anspruch 8, wobei die Membran aus der aus Millipore HF075, HF090, HF120, HF135, HF180 oder HF240 oder Membranen mit vergleichbaren oder gleichwertigen Fließeigenschaften bestehenden Gruppe ausgewählt ist.

10. Fester Träger mit einem daran immobilisierten Fängermolekülkomplex, erhalten mit dem Verfahren nach Anspruch 1 bis 9.

11. Immuntest für den Nachweis von Antikörpern und Antigenen, umfassend einen festen Träger nach Anspruch 10.

12. Immuntest nach Anspruch 11 für den Nachweis von für rheumatoide Arthritis spezifischen Antikörpern.

13. Immuntest nach Anspruch 12, wobei ein cyclisches citrulliniertes Peptid auf dem festen Träger immobilisiert ist.

## Revendications

1. Procédé pour l'immobilisation d'une molécule de capture sur un support solide, dans lequel la molécule de capture est liée de façon covalente à une molécule de biotine pour obtenir une molécule de capture biotinylée et dans lequel la molécule de capture biotinylée est, dans un premier temps, mise en contact avec un composant de liaison pour former un complexe de molécule de capture et ensuite, le complexe de molécule de capture est mis en contact avec le support solide.

2. Procédé selon la revendication 1, dans lequel le composant de liaison est un agent de liaison à affinité élevée choisi dans le groupe constitué de l'avidine, la captavidine, la streptavidine, la neutravidine ou l'hydrazide de streptavidine ou des dérivés de ceux-ci.

3. Procédé selon les revendications 1 ou 2 dans lequel la molécule de capture est une petite molécule.

4. Procédé selon les revendications 1 à 3 dans lequel la molécule de capture est un peptide.

5. Procédé selon les revendications 1 à 4 dans lequel la molécule de capture est un antigène réactif avec des anticorps obtenus à partir de patients souffrant de la polyarthrite rhumatoïde.

6. Procédé selon la revendication 5 dans lequel la molécule de capture est un peptide citrulliné cyclique.

7. Procédé selon les revendications 1 à 6 dans lequel le support solide est une membrane de nitrocellulose.

8. Procédé selon la revendication 7 dans lequel le support solide est une membrane à écoulement rapide.

9. Procédé selon la revendication 8 dans lequel la membrane est choisie dans le groupe constitué de Millipore HF075, HF090, HF120, HF135, HF180 ou HF240 ou des membranes ayant des propriétés d'écoulement comparables ou équivalentes.

10. Support solide avec un complexe de molécule de capture immobilisé sur celui-ci, obtenu par le procédé selon les revendications 1 à 9.

11. Immunodosage pour la détection d'anticorps ou d'antigène comprenant un support solide selon la revendication 10.

12. Immunodosage selon la revendication 11 pour la détection d'anticorps spécifiques pour la polyarthrite rhumatoïde.

13. Immunodosage selon la revendication 12 dans lequel un peptide citrulliné cyclique est immobilisé sur le support solide.
